(19) Europäisches Patentamt
European Patent Office
Office européen des brevets

(11) Publication number: 0 181 061 A1

(12) # EUROPEAN PATENT APPLICATION

(21) Application number: 85306102.6

(22) Date of filing: 28.08.85

(51) Int. Cl.⁴: C 07 C 59/00
G 01 N 33/534

(30) Priority: 07.09.84 GB 8422606

(43) Date of publication of application: 14.05.86 Bulletin 86/20

(84) Designated Contracting States: DE FR GB

(71) Applicant: AMERSHAM INTERNATIONAL plc
Amersham Place
Little Chalfont Buckinghamshire HP7 9NA(GB)

(72) Inventor: Stokes, Malcolm John
16 Wykeham Way
Haddenham Buckinghamshire(GB)

(74) Representative: Pennant, Pyers et al,
Stevens, Hewlett & Perkins 5 Quality Court Chancery Lane
London, WC2A 1HZ(GB)

(54) **Labelling organic molecules with sulphur-35.**

(57) Labelling reagents are derivatives of sulphur-containing amino acids or taurine labelled with an artificially high concentration of S–35, which derivatives include coupling groups reactive with reactive groups of organic molecules to be labelled. Suitable coupling groups include N–succinimidyl or N–sulphosuccinimidyl groups, which are reactive with primary amine groups. Suitable organic molecules are macromolecules particularly proteins and polypeptides. Labelled organic molecules can be prepared having specific activities of more than 50 Ci/mmol, and are suitable for use by autoradiography.

EP 0 181 061 A1

# LABELLING ORGANIC MOLECULES WITH SULPHUR-35

This invention relates to methods and reagents for labelling organic molecules, particularly macromolecules, with sulphur-35 (S-35) to the labelled molecules and to their use in autoradiography. S-35 has a half life of 87.2 days and decays with the production of beta-particles of energy 0.167 MeV. Carrier-free S-35 having a specific activity of about 1500 Ci/mmol is available commercially. A limited range of organic compounds (including sulphur-containing amino acids and taurine) can be produced in labelled form at high specific activity by causing a suitable micro-organism to assimilate a nutrient containing S-35 and generate the desired compound. Polypeptides can be synthesised by a laborious technique involving the use of individual S-35 labelled amino acids. Other compounds can be labelled by isotope exchange techniques or other synthetic methods, but generally only at low specific activity. There is no general S-35 labelling reagent which can be directed at specific desired reactive groups of the compound to be labelled.

By contrast I-125 labelling reagents are well documented and widely used, particularly for labelling proteins and polypeptides. The best known reagent for coupling to amino groups is the Bolton-Hunter reagent (I-125-labelled N-succinimidyl-3-(4-hydroxyphenyl)-propionate,as described in the review of J.J. Langone in Methods in Enzymology, 73, 6, 112-127. Another reagent is tert-butoxycarbonyl-L-(I-125)iodotyrosine N-hydroxy-succinimide ester which has the advantage over the Bolton-Hunter reagent that a derivatized peptide is readily deblocked to form a radio labelled product with the same net charge as the starting material.

I-125 labelled proteins and polypeptides are widely used for analysis by autoradiography. In this application, S-35 labelled proteins and polypeptides can give shorter exposure times and superior resolution (ability to distinguish neighbouring bands or spots of analyte) to their I-125 labelled counterparts. Tritium labelling reagents, e.g. H-3 N-succinimidyl propionate ([$^3$H]NSP) are also well documented. Molecules are labelled with [$^3$H]NSP to specific activities of $<$ 100 Ci/mmol per amino group substituted hence lower sensitivity is achieved than with S-35 labelled molecules. There is a need for general methods and reagents for labelling proteins and polypeptides with S-35. This invention fulfills that need, and in addition provides a method having improved safety compared with existing methods using I-125 labelling reagents.

In one aspect this invention provides a method of labelling an organic molecule having a reactive group, which method comprises providing a labelling reagent which is a derivative of a sulphur-containing amino acid or taurine labelled with an artificially high concentration of S-35 which derivative includes a coupling group reactive with the reactive group of the organic molecule, and bringing together the organic molecule and the labelling reagent under conditions to couple the labelling reagent to the organic molecule by reaction between the reactive group and the coupling group.

In another aspect, the invention provides a labelling reagent which is a derivative of a sulphur-containing amino acid or taurine labelled with an artificially high concentration of S-35 which derivative includes a coupling group reactive with a reactive group of an organic molecule to be labelled.

The method of the invention involves coupling

the labelling reagent to the organic molecule. Although the nature of the organic molecule is not critical, the invention is of particular importance in relation to macromolecules. For example, proteins and polypeptides are widely used in protein blotting applications and can advantageously be labelled by this method. The organic molecule carries a reactive group, but the nature of this is not critical since the only requirement is that it react with the coupling group of the labelling reagent. For proteins and polypeptides, the reactive groups will generally be primary amino or thiol groups. Other possible reactive groups include phenolic hydroxyl, aliphatic hydroxyl, imidazole and guanidyl.

The labelling reagent is a derivative of a sulphur-containing amino acid, for example, cysteine, cysteic acid, cystine, and, particularly, methionine or of taurine. There may be used d- or l-isomers of these amino acids where two or more separate isomers exist, or mixtures of isomers.

The reagent is labelled with an artificially high concentration of S-35, generally at a specific activity of at least 1 Ci/mmol, and this is perfectly adequate for some uses. For other uses including autoradiography, high specific activities of more than 100 Ci/mmol, and preferably at least 1000 Ci/mmol are required. These labelling reagents may be prepared from their respective labelled amino acids, all of which are commercially available at high specific activity. It is an advantage of S-35 labelling reagents based on amino acids that they can be prepared at high specific activity, whereas this is not true of S-35 labelled compounds generally.

The labelling reagent includes a coupling group reactive with the reactive group of the organic molecule to be labelled. Usually, though not always, a

 

carboxylic acid group is modified to provide the coupling group. A preferred coupling group is the N-succinimidyl group that results from the condensation of a carboxylic acid with N-hydroxysuccinimide in the presence of a carbodiimide:

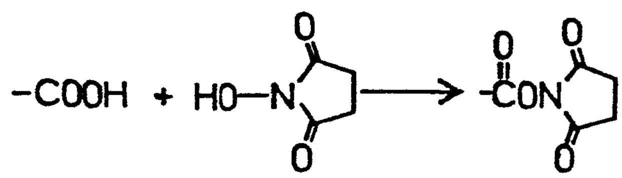

and which readily couples with a primary amine under mild conditions.

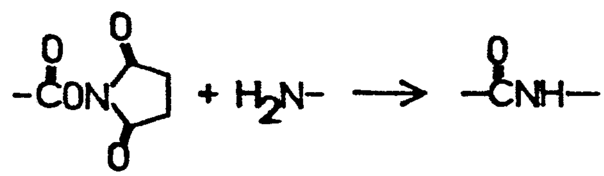

Another similar coupling group, having the advantage of enhanced water solubility is the N-sulphosuccinimidyl group derived from N-hydroxysulphosuccinimide:

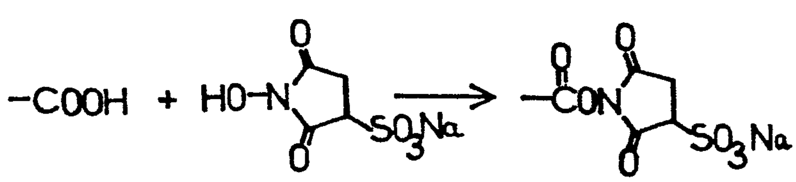

Coupling groups based on maleimide are suitable for reaction with thiol groups. Coupling groups based on hydrazide are suitable for reaction with carbohydrates Many other coupling groups are reported in the literature and can be used. The Radiochemical Centre

Technical Bulletin 79/6, "Protein labelling reagents and references to their applications", gives a non-exhaustive list, with literature references, of labelling reagents with coupling groups suitable for reaction with different reactive groups of proteins and polypeptides.

Amino groups in labelling reagents of this invention generally need to be protected in order to prevent self-condensation. Amine protecting groups are well described in the literature. A book which describes a wide range is "Protective Groups in Organic Synthesis", T.W. Greene, 1981 (J. Wiley & Son) see page 218. Amine protective groups of particular interest are as follows:-

Cleaved under acidic conditions:-

1. *t*-butyl carbamate
2. 1-methyl-1-(1-adamantyl)ethyl carbamate
3. 2-trimethylsilylethyl carbamate

Cleaved under basic conditions:-

1. 9-fluorenylmethyl carbamate
2. 9-(2-sulpho)fluorenylmethyl carbamate
3. 2-phosphonoethyl carbamate
4. 1,1-dimethyl-2-cyanoethyl carbamate
5. pyridinium acetamide

Cleaved under neutral conditions

1. 8-quinolyl carbamate

Cleaved by photolysis

1. *m*-nitrophenyl carbamate

Alternatively, the protecting group can be chosen to be a photoactivatable group such as an aromatic azido group, giving rise to a high specific

activity radioactive photolabile bifunctional reagent. A review of photoaffinity reagents is contained in the book "Photogenerated reagents in biochemistry and molecular biology", H. Bayley, 1983 (Elsevier). Photoactivatable groups react, upon activation, indiscriminately with anything in the vicinity, so that it is not possible to target them for reaction with specific reactive groups. For this reason, we do not contemplate the use of such photoactivatable groups as coupling groups in the labelling reagents of this invention.

Alternatively, an amino group of the amino acid can be modified to form a coupling group which may for example be *N*-succinimidyl or iodoacetyl. In such cases a carboxylic acid group may be protected by esterification.

Preferred labelling agents according to the invention have formulae

a) (Methionine) 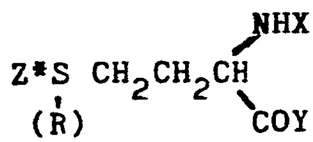

b) (Cysteine) 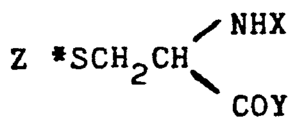

c) (Cysteic acid) 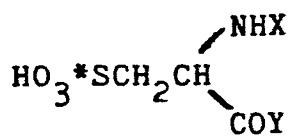

d) (Cystine) 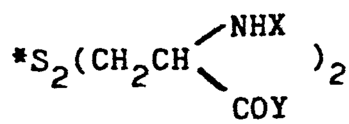

e) (Taurine) $HO_3{*}SCH_2CHNHY$

f) the decarboxylated derivatives of (a) (b) (c) (d) i.e.

i.e. 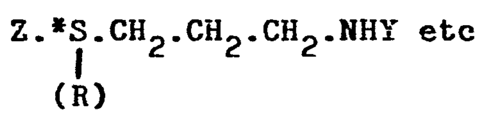

g) the deaminated derivatives of
(a) (b) (c) (d) i.e.
i.e. Z.*S.$CH_2$.$CH_2$.$CH_2$.COY etc.
(R)

where *S means S including an artificially high concentration of S-35

R is either absent or if present means O or $O_2$ or an alkyl group

X is a protecting group such as tertiarybutoxycarbonyl, or which may be photolabile,

Y is a coupling group such as N-succinimidyl, and

Z is an inert,eg alkyl,group or a photolabile group.

These labelled reagents may be prepared by standard chemical routes from the labelled amino acid or taurine. The following reaction diagrams 1 to 5 summarise the chemistry involved. In these diagrams the following abbreviations have been used.

*S means S with an enhanced concentration of S-35.
BOC means tert-butoxycarbonyl
NHS means N-hydroxysuccinimide, or (when forming part of a labelled compound) N-succinimidyl
BOC-ON means 2-(tert-butoxycarbonyl oxyimino)-2-phenylacetonitrile
DCC means dicyclohexylcarbodiimide
per TFA means trifluoroperacetic acid
MCPBA means m-chloroperbenzoic acid

These summaries are not intended to be exhaustive. Thus in diagram 1, any reagent based on methionine could be oxidised either to the sulphone or to the sulphoxide, although these reactions are only shown for one reagent. Further in each of diagrams 1, 2, 3 and 4, any amino acid could be either decarboxylated or deaminated, although this has not been shown. Decarboxylation or deamination would normally be

performed on the labelled amino acid before derivatization. A labelling reagent based on such a decarboxylated or deaminated amino acid would contain a coupling group but normally no protecting group.

**DIAGRAM 1** **PRODUCTS BASED ON METHIONINE**

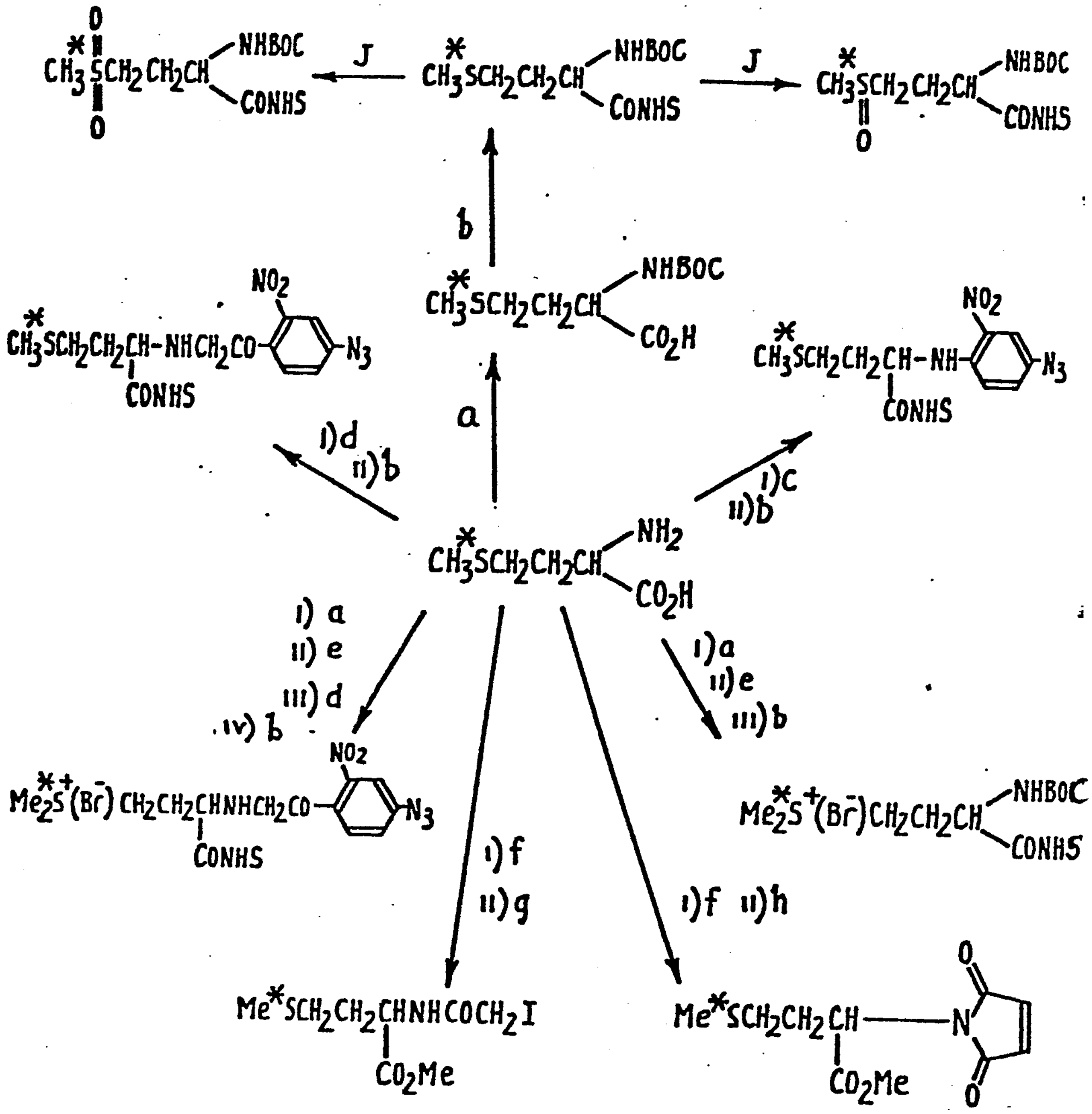


a) BOC-ON/$Et_3N$ b) NHS/DCC c) 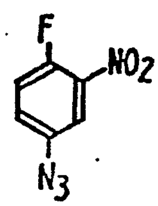 d) 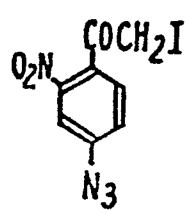

e) MeBr f) $M^+$/MeOH g) $ClCOCH_2Cl$/NaI h) 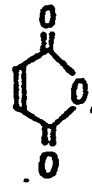/DCC J) $(n-C_4H_9)_4N^+IO_4^-$

## DIAGRAM 2 PRODUCTS BASED ON METHIONINE SULPHOXIDE & METHIONINE SULPHONE

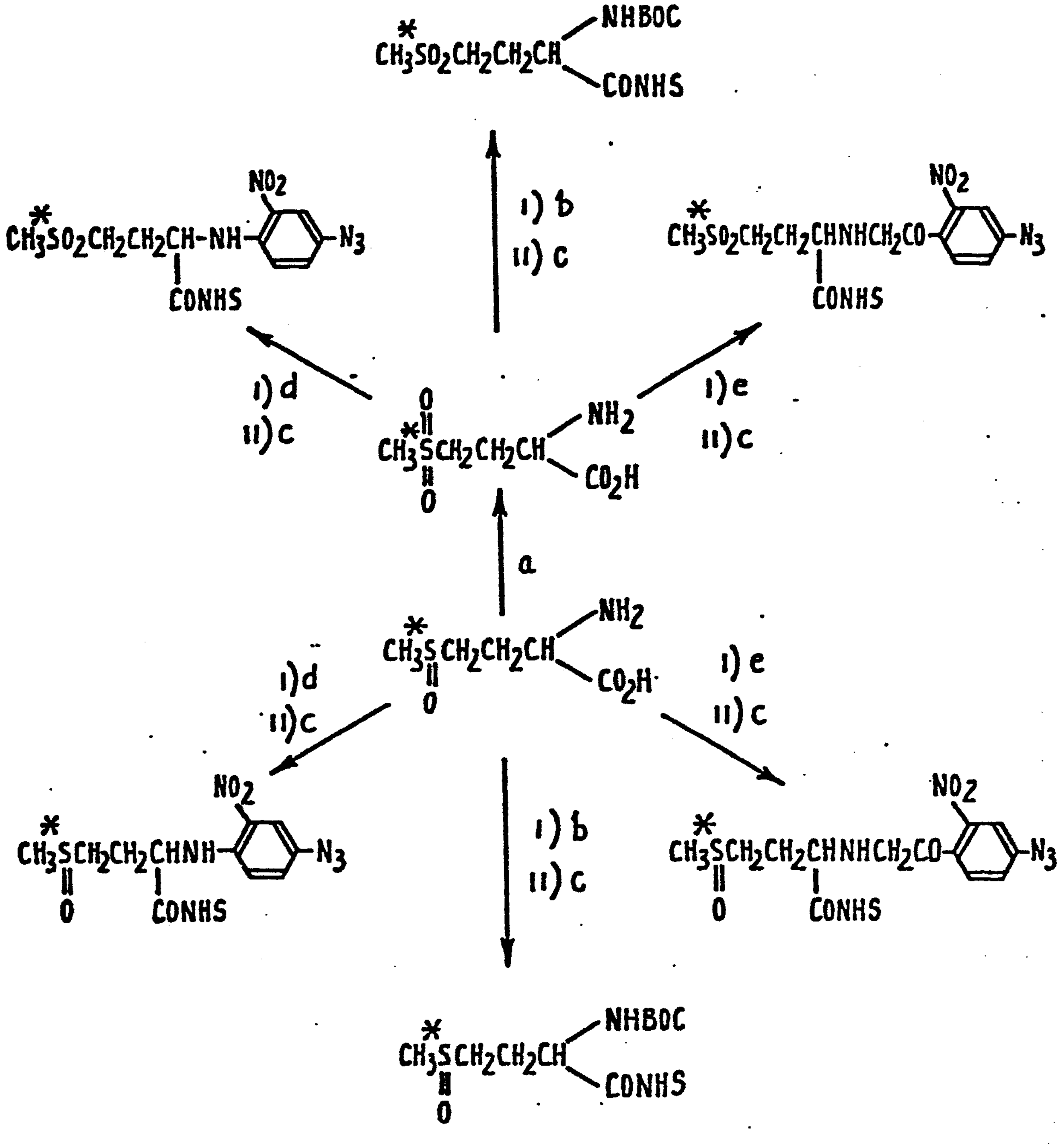


a) $CF_3CO_3H$ b) BOC-ON/$Et_3N$ c) NHS/DCC d) 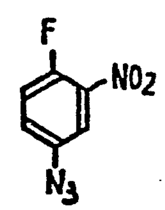 e) 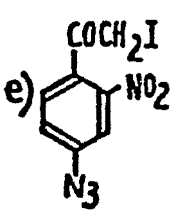

DIAGRAM 3 PRODUCTS BASED UPON CYSTEINE

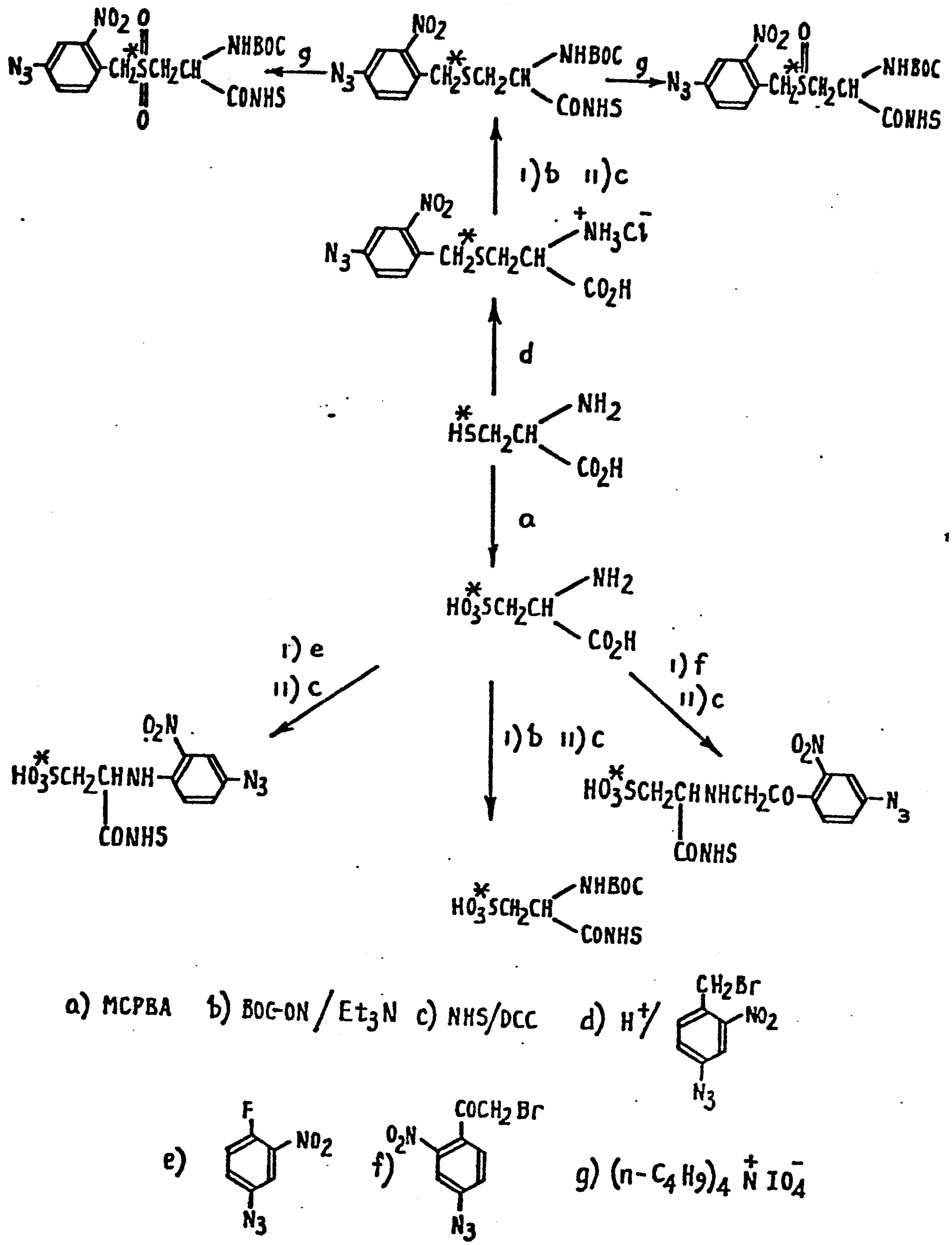

**DIAGRAM 4 PRODUCTS BASED UPON CYSTINE**

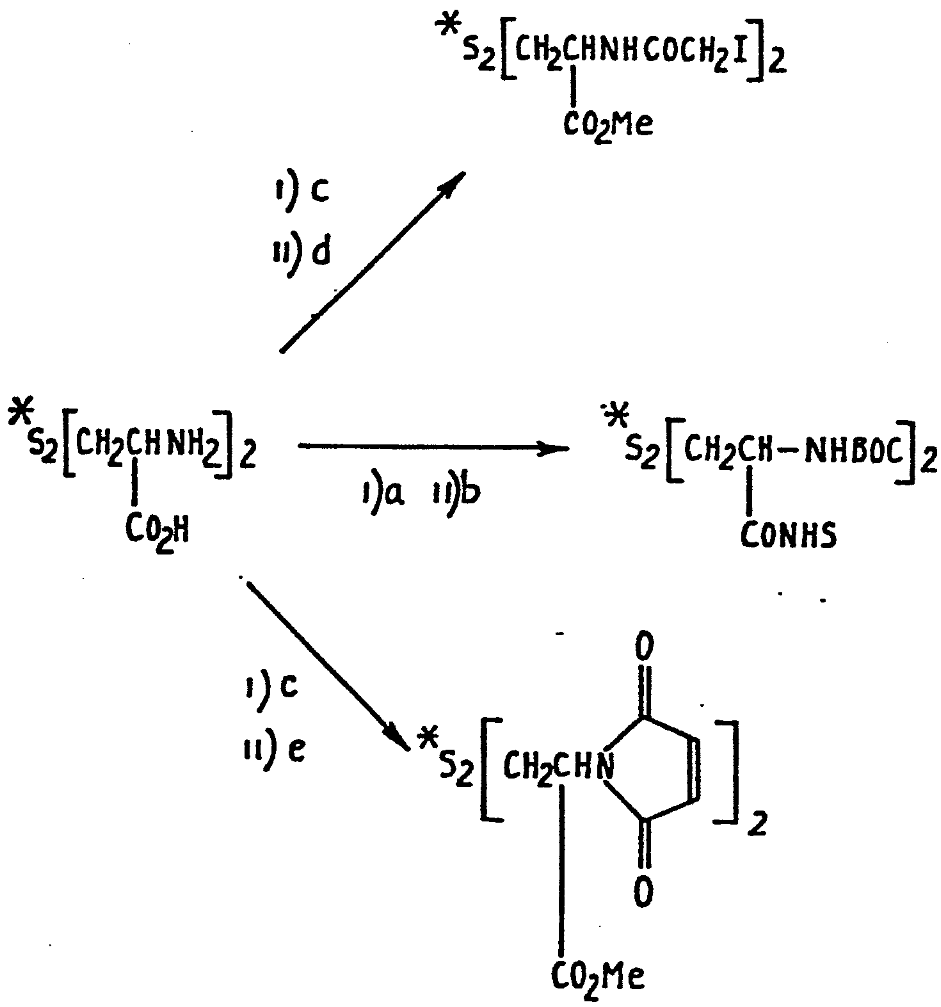


a) BOC-ON/$Et_3N$ b) NHS/DCC c) $H^+$/MeOH d) $ClCOCH_2Cl$/NaI

e) 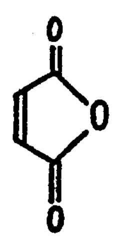 / DCC.

DIAGRAM 5 PRODUCTS BASED UPON TAURINE

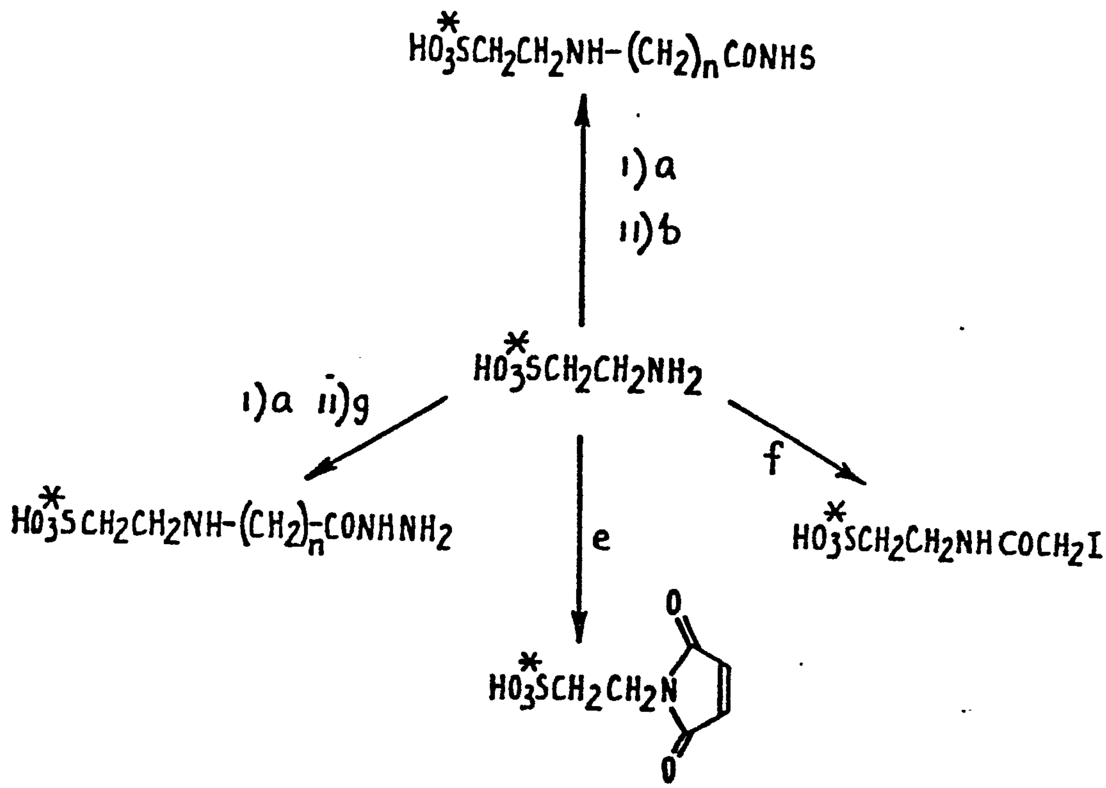


a) $Br(CH_2)_nCO_2H$ b) NHS/DCC c) 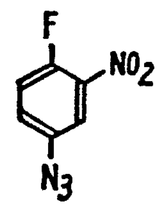

d) 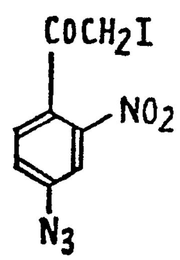 e) 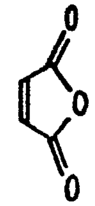 /DCC f) $ClCOCH_2Cl/NaI$

g) $N_2H_4$

The nature of the organic molecule to be labelled by this invention is not critical. Classes of macromolecules which can be labelled in this way include nucleic acids, oligosaccharides, polysaccharides and heteropolysaccharides. But the invention is likely to be of particular importance in the labelling of proteins and polypeptides, which terms are defined broadly to include glycoproteins, lipoproteins, nucleoproteins, mucoproteins, haemoproteins and lectins. Other organic molecules which can be labelled with advantage include biotin analogues such as biocytin and analogues of the calcium channel blockers iodopine and azidopine and related compounds.

The method of this invention involves reacting an organic molecule having a reactive group, particularly a macromolecule such as a protein or peptide having a series of reactive groups, with the labelling reagent. The chemistry of this reaction is standard and depends on the nature of the coupling group of the labelling reagent.

For labelling proteins and peptides, this reaction in similar to that undergone by the Bolton-Hunter reagent. But the Bolton-Hunter reagent is perceived to have a disadvantage in that it may decompose to form very small amounts of free volatile $^{125}I_2$. With the diiodo compound, this disadvantage is certainly very real. For this reason, it is customary to use charcoal traps during evaporation of the benzene prior to use. The S-35 labelling reagents of this invention do not suffer from this disadvantage.

By way of example, the reaction of lysine epsilon $NH_2$ groups of protein with a labelling reagent based on methionine can be shown as

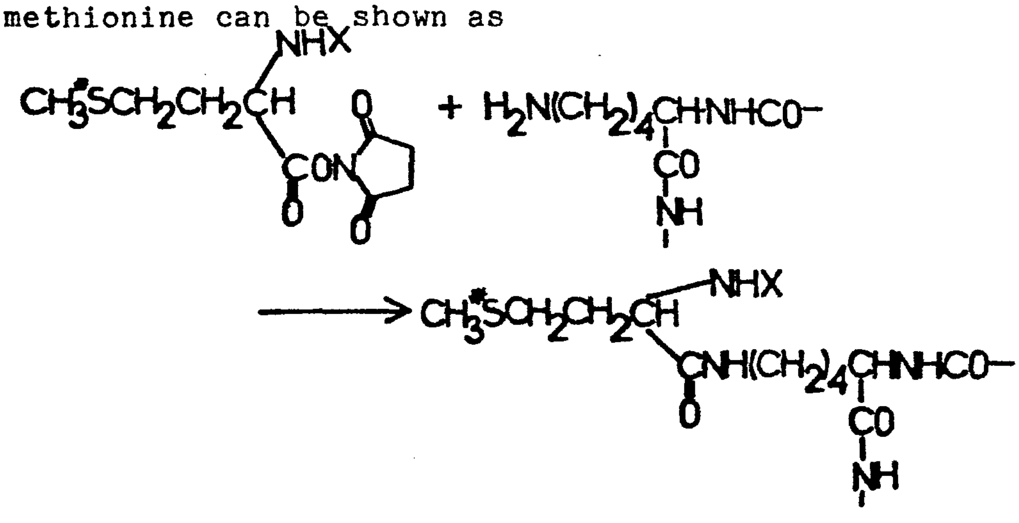

At this point, the protecting group X can if desired be left in position. Or it can be removed to regenerate a primary amino group, thereby retaining the original charge on the molecule. Where X is a photoactivatable group, it can be activated so as to cross-link the labelling reagent/protein conjugate to another protein molecule for example a receptor.

In another aspect, this invention includes macromolecules such as proteins and polypeptides labelled with S-35 at a specific activity of at least 50 Ci/mmol, preferably at least 100 Ci/mmol. The method of this invention permits for the first time labelling of macromolecules with S-35 at such high specific activities.

Products which we have labelled by the method of this invention include Protein A, anti-mouse Ig whole antibody, anti-rabbit Ig whole antibody and streptavidin. Other products which can be labelled similarly include $F(ab')_2$ fragments, protein molecular weight markers and lectins.

These products are characterized by having side chains which have been modified by reagents derived from sulphur-containing amino acids including an artificially high proportion of S-35. Many of these products are new materials in their own right and are included as such in the scope of this invention. The labelled products generally have specific activities of at least 1 Ci/mmol, preferably at least 50 Ci/mmol, depending on the number of reactive groups in the molecule and the molar ratio of reagent to substrate.

The S-35 labelled products are particularly valuable for applications involving autoradiography. In this technique a photographic film is placed directly over the radiolabelled material. Silver halide crystals in the emulsion respond to the beta-particles emitted by S-35. Film blackening increases

with the number of beta particles emitted until saturation is reached.

The reagents described are particularly useful in blotting experiments in which proteins are transferred from a polyacrylamide gel to the surface of a membrane (e.g. nitrocellulose) and then detected immunochemically. The technique is reviewed by J.M. Gershoni et al in Analytical Biochemistry 131 1-15 (1983).

The most widely used radioactive detection agents for protein-blotting are I-125 labelled proteins and polypeptides. However, S-35 labelled proteins and polypeptides have several advantages, as follows:-

a) S-35 has a longer half-life (87.2 d) than does I-125 (60 d).

b) Labelling of proteins and polypeptides with I-125 using the Bolton-Hunter reagent involves, as noted above, some slight hazard from volatile $^{125}I_2$.

c) The gamma-rays produced by I-125 tend to pass straight through photographic film so that only a small proportion of their energy is captured and recorded by the film, most being wasted. This problem can to some extent be overcome by the use of an intensifying screen, with the film sandwiched between the screen and the sample,but the resulting improvement in sensitivity is obtained at the expense of resolution.Overall, our work indicates that improved sensitivity and improved resolution can be obtained at the same time in autoradiography work by using S-35 labelled compounds in place of I-125 labelled ones.

In view of these advantages, it is perhaps surprising that S-35 labelled proteins and polypeptides have not supplanted I-125 labelled ones when autoradiography is used as the detection method. The reason is believed to be the lack of a general S-35 labelling reagent and the resulting difficulty in preparing the labelled compounds at high specific

activity. The labelling reagents and the preparative method of this invention should overcome this problem.

S-35 labelled compounds and I-125 labelled compounds can be used together with advantages in a double autoradiographic technique. A sample is provided in a suitable form for autoradiography, one component is labelled with S-35 and another is labelled with I-125. On the sample are laid successively a first photographic film, a sheet of paper and a second photographic film. The first photographic film records (radioactive) emissions by both S-35 and I-125; The second film records emission of I-125 only. Thus the location and concentration of two proteins (or other species) in the sample can be determined in a single autoradiography experiment.

The labelling reagent can be used to label receptor ligands for studies of the autoradiographic distribution of receptors in tissue sections. H-3 anc I-125 are currently being used for studies of this nature, for example, R. Quirion et al Life Sci 33, 227-230 (1983) and C. W. Shults et al Peptides 3 1073-1075 (1983). These receptors are present in very low quantities and using $^{3}H$ ligands autoradiographs need to be exposed for 1-3 months.

The higher specific activity and higher beta energy of S-35 (0.167 MeV for S-35 compared with 0.0186 MeV for H-3) will allow a great reduction in exposure time if a S-35 labelled ligand is used. Intensifying screens are frequently used in the detection of I-125 with a resultant decrease in resolution. Such screens are unnecessary for S-35 so there is no loss in resolution.

The following Examples illustrate the invention.

Example 1 describes the preparation of a labelling reagent based on methionine.

Example 2 describes a method of labelling a protein by reaction with the labelling reagent of Example 1.

Example 3 describes a double-labelling experiment using S-35 and I-125 labelled proteins.

Example 4 describes experiments which demonstrate the improved resolution and shorter exposure times that can be achieved in autoradiography by using S-35 labelled proteins in place of their I-125 counterparts.

## EXAMPLE 1

**$^{35}$S-tert-Butoxycarbonylmethionine N-Hydroxysuccinimide ester ($^{35}$SLR) $^{35}$-Sulphur-Labelling-Reagent**

$^{35}$SLR is prepared from [$^{35}$S] methionine in two steps. The amino group of methionine is protected with the tert-butoxycarbonyl group (BOC), and the acid group of methionine is activated by condensation with N-hydroxysuccinimide (NHS) using dicyclohexylcarbodiimide (DCC). The product is purified by HPLC.

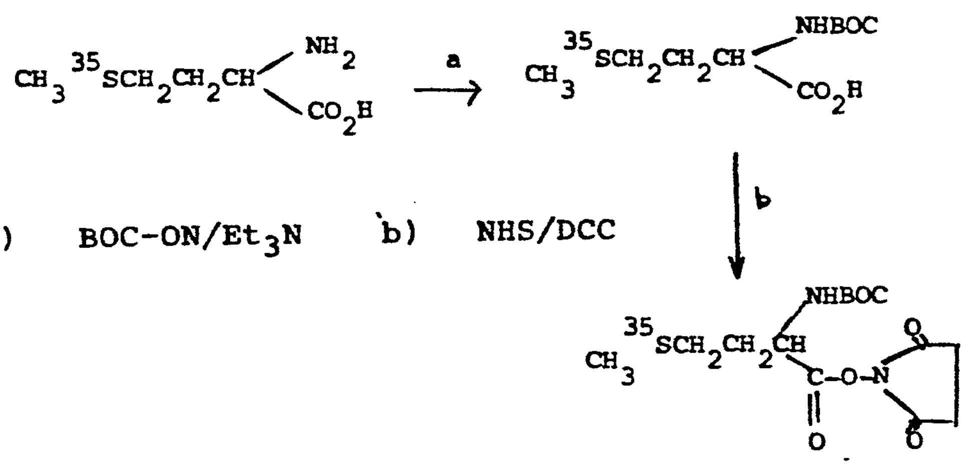


Method

To a stirred aqueous solution of $^{35}$S-methionine (SJ204, 2 Ci, 50 mCi/ml, specific activity (SA)>1000 Ci/mmol in dioxan) (30 ml) is added triethylamine (100 $\mu$l; 234

μl/ml in dioxan) and 2-(tert-butoxycarbonyl oxyimino)-2-phenylacetonitrile (BOC-ON, 160 μl; 500 mg/ml in dioxan). The reaction is stirred at room temperature for 3 h. The majority of the dioxan is removed under vacuum on a rotary evaporator and the aqueous layer extracted with ethyl acetate (2 x 10 ml). Ethyl acetate (10 ml) and citric acid solution (1.4 ml; 5%) is added to the aqueous layer, and the mixture stirred at room temperature for 10 min. The organic and aqueous layers are separated and the aqueous layer extracted a further four times with ethyl acetate (4 x 10 ml). The ethyl acetate is completely removed under vacuum and replaced with dry dioxan (40 ml). N-hydroxysuccinimide (125 μl; 400 mg/ml in dry dioxan) is added to the stirred reaction mixture followed by dicyclohexylcarbodiimide (160 μl; 500mg/ml in dioxan). The reaction is stirred at room temperature overnight. The precipitated dicyclohexyl urea is removed by filtration and the remaining solution purified by HPLC. The product was characterized by HPLC and TLC comparisons with an authentic (unlabelled) sample, which is commercially available, by its performance in a number of labelling reactions as discussed below.

Yield ~ 25%. SA> 1000 Ci/mmol.

An oxidized form of the reagent can be obtained using tetrabutylammonium periodate, giving a more stable product that is soluble in water. Labelling reagents derived from cysteine, cysteic acid and cystine may be obtained similarly.

## EXAMPLE 2

### $^{35}$S-Labelled Proteins

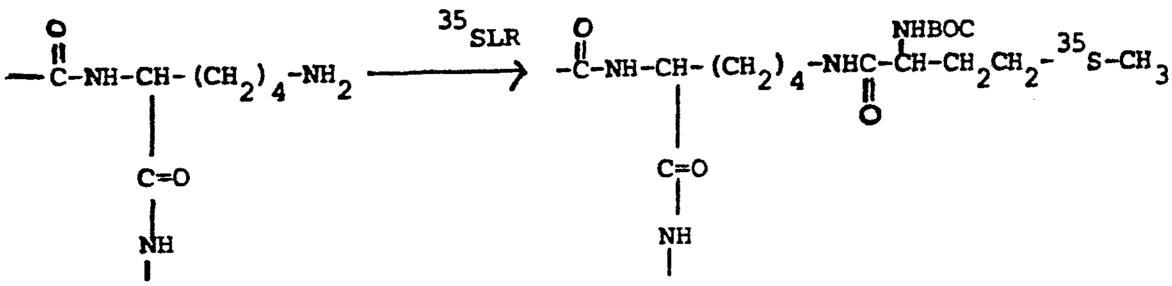


Method - $^{35}$SLR (10 m Ci; 1 mCi/ml in benzene) is evaporated to dryness by a slow stream of dry nitrogen. The sides of the reaction vessel are carefully washed with dry benzene (100 µl) which is then evaporated under a stream of nitrogen. The reaction vessel is cooled in ice and the protein (10 mg/ml in phosphate buffer pH 8.6) added. After 30 min. the reaction is terminated with aqueous tyrosine solution (100 µl; 1.8 mg/ml). Purification of the product is achieved by chromatography on Sephadex G25.

Yield 20-50% SA 200-1000 Ci/mmol (depends on $NH_2$ content of the protein and the molar ratio of protein: $^{35}$SLR used).

By means of this reaction were prepared S-35 labelled
Protein A,
anti-mouse Ig whole antibody,
anti-rabbit Ig whole antibody,
streptavidin.

The products were tested by their performance in protein blotting experiments and compared with the known $^{125}I$-labelled products.

EXAMPLE 3

Double Labelling

$^{35}S$-and $^{125}I$-labelled Protein A and species-specific second antibodies provide the opportunity of detecting and distinguishing two antigens in the same sample on a protein blot in a single autoradiography step using two films.

Human brain homogenate was electrophoresed on an SDS-polyacrylamide gel and transferred to nitrocellulose. The blot was probed with a monoclonal antibody against $\beta$-tubulin, followed by $^{35}S$-labelled anti-mouse Ig (5$\mu$Ci, specific activity$\sim$240 Ci/mmol) and then probed again with a monoclonal antibody against $\alpha$-tubulin followed by $^{125}I$-labelled anti-mouse Ig (5 $\mu$Ci; SA$\sim$1600 Ci/mmol).

The blot was then autoradiographed by placing a film directly over the blot detecting $^{35}S$ and $^{125}I$(and therefore $\alpha$ and $\beta$ b-tubulin) and a second film on top of the first, separated by a paper sheet; which only detects $^{125}I$ and therefore only $\alpha$-tubulin.

EXAMPLE 4

Shorter exposure time and resolution

To demonstrate the advantages in terms of higher resolution and shorter exposure times using a $^{35}S$-labelled protein, $^{35}S$-labelled anti-rabbit Ig and $^{125}I$-labelled anti-rabbit Ig were compared in a system using human brain homogenate.

Human brain homogenate (200 ug protein) was electrophoresed on a SDS-polyacrylamide gel and the

proteins transferred to nitrocellulose and probed with rabbit antiserum to human cerebrum. The antigen antibody complexes were detected using either $^{35}S$-labelled anti-rabbit Ig (5μCi; SA~240 Ci/mmol) or $^{125}I$-labelled anti-rabbit Ig(5 μCi; about 1600 Ci/mmol). Bands are more clearly resolved when the $^{35}S$-labelled protein is used.

As little as 4 hours exposure time was sufficient, using $^{35}S$-labelled anti-rabbit Ig to obtain clearly resolved bands whereas 16 hours was required using the $^{125}I$-labelled reagent and an intensifying screen

CLAIMS

1. A method of labelling an organic molecule having a reactive group, which method comprises providing a labelling reagent which is a derivative of a sulphur-containing amino acid or taurine labelled with an artificially high concentration of S-35 which derivative includes a coupling group reactive with the reactive group of the organic molecule, and bringing together the organic molecule and the labelling reagent under conditions to couple the labelling reagent to the organic molecule by reaction between the reactive group and the coupling group.

2. A method as claimed in claim 1, wherein the organic molecule is a macromolecule selected from proteins and polypeptides.

3. A method as claimed in claim 2, wherein the reactive group is the epsilon amino group of lysine or the primary amino group of a N-terminal amino acid.

4. A method as claimed in any one of claims 1 to 3, wherein the labelled organic molecule has a specific activity of at least 50 Ci/mmol.

5. A method as claimed in any one of claims 1 to 4, wherein the organic molecule is a protein selected from Protein A, anti-mouse whole antibody, anti-rabbit whole antibody, and streptavidin.

6. A labelling reagent which is a derivative of a sulphur-containing amino acid or taurine labelled with an artificially high concentration of S-35 which derivative includes a coupling group reactive with a reactive group of an organic molecule to be labelled.

7. A labelling reagent as claimed in claim 6, wherein the sulphur-containing amino acid is selected from methionine, methionine sulphoxide, methionine sulphone cysteine, cysteic acid and cystine.

8. A labelling reagent as claimed in claim 6 or claim 7, wherein the coupling group is an N-succinimidyl or N-sulphosuccinimidyl group.

9. A labelling reagent as claimed in any one of claims 6 to 8, wherein any amino groups are protected by a protecting group.

10. A labelling reagent as claimed in claim 9, wherein the protecting group is photoactivatable to make the reagent bifunctional.

11. A labelling reagent as claimed in any one of claims 6 to 9, having one of the formulae:-

a) (Methionine) 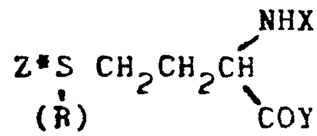

b) (Cysteine) 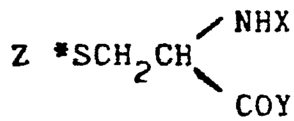

c) (Cysteic acid) 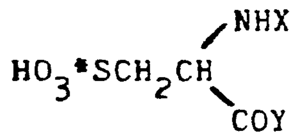

d) (Cystine) 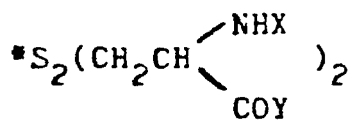

e) (Taurine) $HO_3{}^*SCH_2CHNHY$

f) the decarboxylated derivatives of (a) (b) (c) (d) i.e.
i.e. 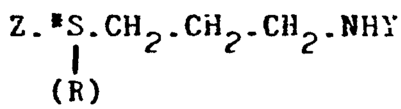 etc.

g) the deaminated derivatives of (a) (b) (c) (d) i.e.
i.e. 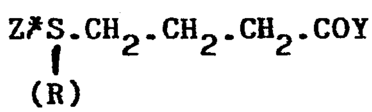 etc.

where *S means S including an artificially high concentration of S-35,

R is either absent or if present means 0 or $0_2$ or an alkyl group,

X is a protecting group,

Y is a coupling group, and

Z is an inert group or a photolabile group.

12. A macromolecule selected from proteins and polypeptides labelled with S-35 at a specific activity of at least 50 Ci/mmol.

13. A macromolecule selected from proteins and polypeptides labelled with S-35, wherein the labelling group is the residue of a labelling reagent as claimed in any one of claims 6 to 11.

14. A method of detecting molecules which have been blotted on to a solid membrane, by contacting the membrane with a radioactively labelled material reactive with the molecules to be detected, removing unreacted labelled material, and subjecting the membrane to autoradiography, characterized in that the labelled material is labelled with S-35.

15. A method of detecting receptors in a tissue slice, by contacting the tissue slice with a radioactively labelled material reactive with the receptors to be detected, removing unreacted labelled material, and subjecting the tissue slice to autoradiography, characterized in that the labelled material is labelled with S-35.

16. A method as claimed in claim 14 to claim 15, wherein the radioactively labelled material is a macromolecule as claimed in claim 12 or claim 13.

European Patent Office

# EUROPEAN SEARCH REPORT

0181061
Application number

EP 85 30 6102

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-1 224 192 (COMMISSARIAT A L'ENERGIE ATOMIQUE)<br>* Whole document *<br>--- | 1,6,7 | C 07 C 59/00<br>G 01 N 33/534 |
| Y | FR-A-1 566 692 (MINISTERUL INDUSTRIEI CHIMICE)<br>* Whole document *<br>----- | 1,6,7 | |
| | | | TECHNICAL FIELDS SEARCHED (Int. Cl.4) |
| | | | G 01 N |

The present search report has been drawn up for all claims

| Place of search | Date of completion of the search | Examiner |
|---|---|---|
| THE HAGUE | 11-12-1985 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82